Europäisches Patentamt

**European Patent Office**

**Office européen des brevets**

(19)

(11) Publication number: **0 072 255**
A2

# EUROPEAN PATENT APPLICATION

(12)

(21) Application number: **82304236.1**

(22) Date of filing: **11.08.82**

(51) Int. Cl.³: **A 61 B 5/10, A 61 B 9/00**

(30) Priority: **11.08.81 GB 8124448**

(43) Date of publication of application: **16.02.83 Bulletin 83/7**

(84) Designated Contracting States: **CH DE FR GB LI SE**

(71) Applicant: **ROLLS-ROYCE LIMITED, 65 Buckingham Gate, London, SW1E 6AT (GB)**

(72) Inventor: **Cornish, Douglas Cecil, 31 Lime Tree Avenue, Rugby (GB)**

(74) Representative: **Leaman, Keith, Rolls-Royce Limited P.O. Box 3 Filton, Bristol BS12 7QE (GB)**

(54) An aesthesiometer.

(57) An aesthesiometer for determining the cutaneous sensitivity of a person comprises a vibrator 12 for vibrating a member 14 at a known frequency and amplitude. The patient places his finger on the member 14 and the amplitude of the vibration is varied from a minimum value until the patient just feels the vibration whereupon the amplitude is noted using an accelerometer 30. The test is carried out at a number of known frequencies noting the amplitude at which the patient just feels the vibration. The amplitude may be varied to increase and decrease the amplitude cyclically and in this case the threshold levels at which the patient just feels, or loses the sense of feeling, the vibrations is noted.

-1-

## AN AESTHESIOMETER

This invention relates to apparatus for monitoring the cutaneous sensitivity of a person and in particular to apparatus for determining the threshold of cutaneous sensitivity of a person.

Such an apparatus would be useful, for example, in detecting or monitoring diminution of cutaneous sensitivity such as occurs with persons liable to experience Raynaud's Phenomenon.

Raynaud's Phenomenon is defined as the intermittent constriction, or spasms, of the arterioles and veins of a person's extremities which results in colour change such as pallour, cyanosis, or both. Raynaud's Phenomenon, commonly precipitated by exposure to cold, and stress, may occur primarily as in Raynaud's Disease (e.g. "constitutional white finger") or secondary to a number of conditions and diseases. Raynaud's Disease is familial, and about 10% of the population of the United Kingdom of Great Britain are prone to experience the disease. Of this 10% of population, the disease is about ten times more prevalent in females than in males. The onset of the disease usually occurs in age groups in the first and second decades, and about 60% of those liable to experience the disease do so by the age of 30.

Despite these trends, it is known that in some occupations, the occurance of Raynaud's Disease amongst men is higher than the national statistics predict. It is well known that in those occupations involving the handling of vibrating equipment, tools, or workpieces, the vibration can induce Raynaud's Disease (known as "Vibration White Finger" or"V.W.F."). Such high risk occupations are, for example, those involving swaging, grinding, fettling (also known as wibbling) polishing drilling, hand rivetting, chain saw operating and vibro-etching.

The degree of risk to individuals in these occupations depends upon the amplitude, duration and frequency of the vibration and also upon the exposure time and susceptibility of the individual concerned, and vibrations of low frequencies, e.g. 2 to 16 Hz can be quite damaging.

The diminution of cutaneous sensitivity as a precursor of V.W.F. has long been established and it has been speculated that stiffness or cutaneous thickening, change in plasticity of skin and sub-cutaneous callus formation at the fingertips might facilitate V.W.F. Persons predisposed to V.W.F. also have much thicker skin than those not so predisposed.

Previous methods of monitoring sub-cutaneous sensitivity have involved the patient moving his finger along a groove, or ridge, the depth of which varies along its length,and relying on the patient's response to the sense of depth to provide an indication of digital sensitivity. With those earlier methods the patient could intentionally or unintentionally introduce bias and render the results unreliable. For example, the earlier tests required the patient to follow the ridge, or groove visually as the finger is moved along the ridge or groove, this enabled the patient to anticipate the point along the ridge or groove where he could feel the ridge.

-3-

There is, therefore, a need for an apparatus to monitor the cutaneous sensitivity of those persons in high risk occupations in order to identify those suffering from, or those likely in later life to suffer from, Vibration White Finger. Those individuals at risk may then be moved to tasks where they do not handle vibrating equipment and tools.

The invention as claimed enables one to determine a person's threshold level of cutaneous sensitivity using vibrations at preselected frequencies and amplitudes and monitoring the amplitude of the vibration at which the person can just feel the vibrations, and makes it more difficult for the patient to influence the results of the test by relying solely on the patient's sense of feel.

The invention will now be described by way of examples only, with reference to the accompanying drawings in which:

Figure 1 shows schematically apparatus constructed in accordance with the present invention.

Figure 2 illustrates schematically an electronic control system for use with the apparatus of Figure 1 enabling the amplitude of vibration of member 14 to be controlled and monitored.

Figure 3 illustrates a modification to the control system of Figure 2 enabling the amplitude of the vibration of the member 14 to be increased and decreased cyclically, and,

-4-

Figures 4 and 5 are graphs of the responses of a number of patients showing the threshold levels of amplitude of vibration at which the patient just feels the vibration. The graphs are plots of amplitude of vibration (in inches x $10^{-n}$) against various frequencies of vibration.

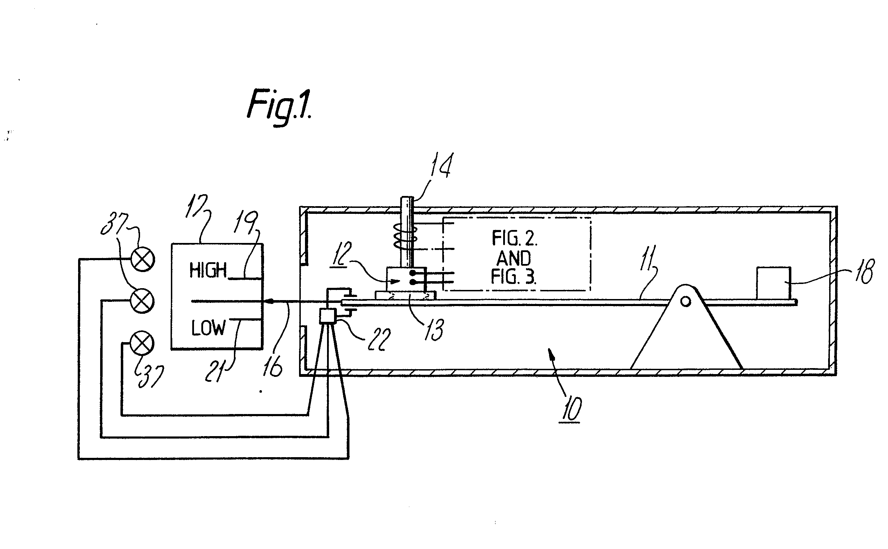

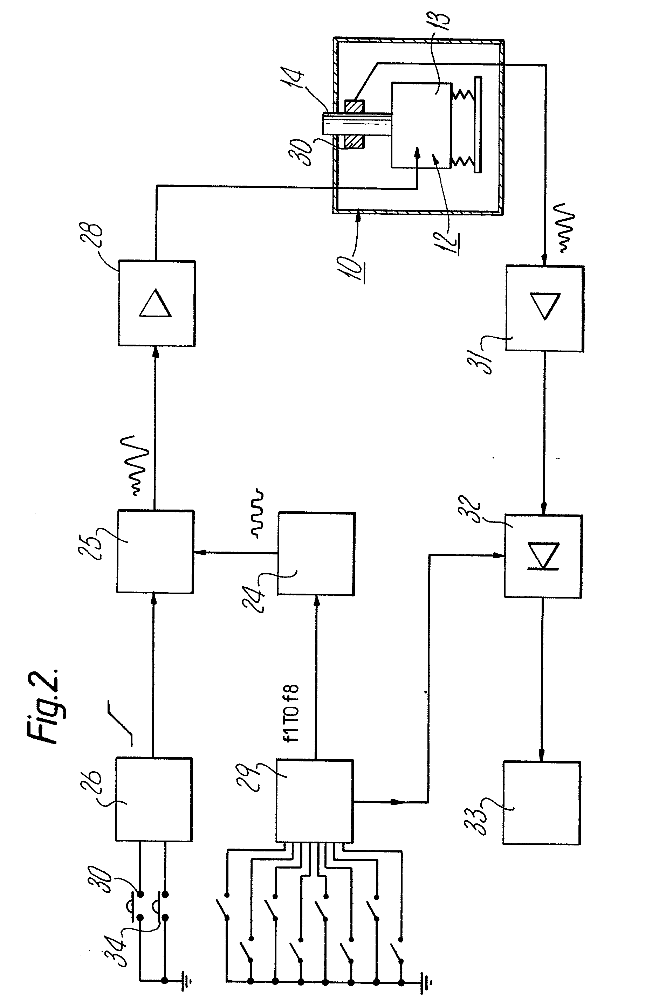

Referring to Figures 1 and 2 the apparatus 10 comprises a balanced beam 11, on one side of which is mounted a vibrator 12, comprising a moving coil vibration generator 13 which is a Ling Dynamics Series 101, 3 ohm vibration generator. The generator 13 is provided for vibrating a member 14 at a controlled frequency and amplitude. A miniature piezo electric accelerometer 15 made by Bruel and Kjaer (Type 4339) is provided for measuring the amplitude of the vibrations of the member 14. The generator 13 is mounted on the beam 11 in rubber mountings or on coil springs. The beam 11 is provided with a pointer 16 which registers with a fixed scale 17 and the beam has a counter weight 18 which biasses the beam to a position indicated by the top line 19 on the scale 17. In use the patient is required to place his finger on the member 14 and push the beam 11 down with a predetermined force of about 0.25 oz. ( 7g. ) to bring the pointer in line with the datum 20. Too much pressure on the member 14 moves the beam towards the lower line 21.

A microswitch 22 is provided alongside the beam 11 so that it is switched to the on position only when the correct force is applied to the beam 11 (i.e. when pointer 16 aligns with the line 20). Indicator lights 17 may be provided to indicate to the patient whether the beam is too high, too low or in the correct position.

-5-

Referring to Figure 2, the vibration generator 13 is driven by an oscillator 24, the output signal of which is a continuous sine wave of uniform amplitude. The output signal of the oscillator 24 is fed to a voltage controlled amplifier 25, the gain of which is controlled by a ramp generator 26. The output signal of the amplifier 25 is a continuous sine wave of increasing amplitude, and this signal is amplified by a power amplifier 28 to drive the moving coil generator 13 and thereby vibrate the member 14.

The oscillator 24 is tunable over a range of frequencies by means of a frequency control circuit 29. The frequency control circuit 29 is switchable to eight discrete frequency channels. The chosen frequencies are 15 Hz, 30 Hz, 60 Hz, 120 Hz, 240 Hz, 360 Hz, 480 Hz and 720 Hz.

The vibration of member 14 is monitored by a miniature piezo electric accelerometer manufactured by Bruel and Kjaer - Type 4339. The output signal of the accelerometer 16 is a sine wave, the amplitude of which is a measure of the distance moved by the member 14. The output signal of the accelerometer 30 is amplified by a charge amplifier 31 and fed to an input of a RMS to DC converter 32.

The output signal of the frequency control circuit 29 is also fed to an input of the converter 32, and the converter 32 incorporates a comparitor circuit which compares the frequency of the output signal of the frequency control circuit 29 with that of the output signal of the accelerometer 30. The output signal of the converter 32 is fed to an input of a digital voltmeter 33 which is scaled to present the voltage level as the amplitude of the vibration of member 14.

In operation, the patient places his finger on the member 14 and pushes the beam 11 down with a force of about 0.25 oz ( 7g. ) until the pointer 16 aligns with the datum 20. The doctor selects a frequency which is unknown to the patient and the patient switches on the switch 34.

The member 14 is vibrated and the ramp generator increases the amplitude gradually. As soon as the patient feels the vibration the patient releases the switch and the amplitude of the vibration displayed on the voltmeter is noted. The test is repeated for different frequencies and the patient's response to the vibration is noted by recording the threshold level of the amplitude that the patient can just feel.

If desired the ramp generator can be set to control the amplitude of the vibration so that at each selected frequency the amplitude is decreased gradually from a predetermined maximum value. In this case the patient switches the apparatus on by switch 34 and immediately feels the vibration. The amplitude continues to fall gradually until the patient no longer feels the vibration whereupon the patient releases the switch 34 to stop the apparatus and the amplitude of the vibration is noted.

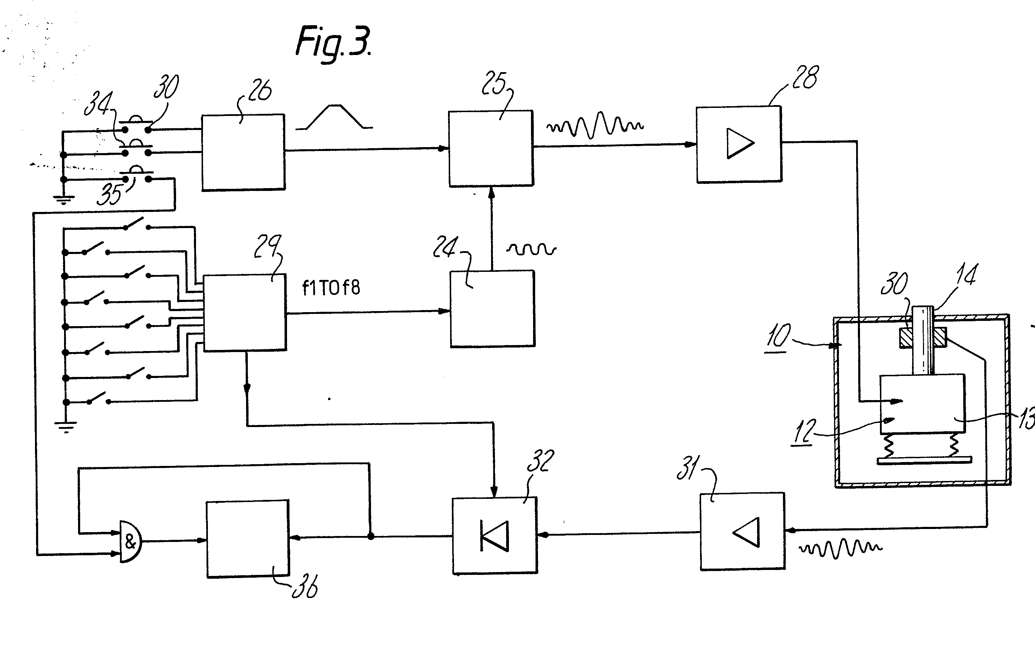

In yet a further modification of the apparatus, the ramp generator may be designed to vary the amplitude cyclically. For example, for each selected frequency the amplitude of the vibration of member 14 may be increased gradually towards a maximum value and then decreased. The patient switches the apparatus on with switch 34 and the

apparatus runs continuously. The patient is then provided with a second switch 35 which operates a recording device 36 see Figure 3. The amplitude increases gradually and the patient opens the switch 35 immediately he feels the vibration and the amplitude of the vibration at this point in time is recorded. The amplitude then continues to increase to a maximum value whereupon the amplitude is gradually decreased until the patient can no longer feel the vibration. The patient then operates switch 35 to operate the recording device to record the amplitude at which he just loses the sense of feel of the vibration. The apparatus may be provided with an automatic frequency selection circuit which responds to the operation of the switch 35 by the patient to select different frequencies.

The apparatus may be operated randomly. That is to say, the doctor may select the same frequency a number of consecutive times, operate the apparatus only on the part of the wave form where the amplitude is increasing, or decreasing, or select different frequencies at random.

The recording device may be a visual display, e.g. on an oscilliscope, or digital or analogue voltmeter; a video recorder; a pen recorder; or a magnetic tape recording system.

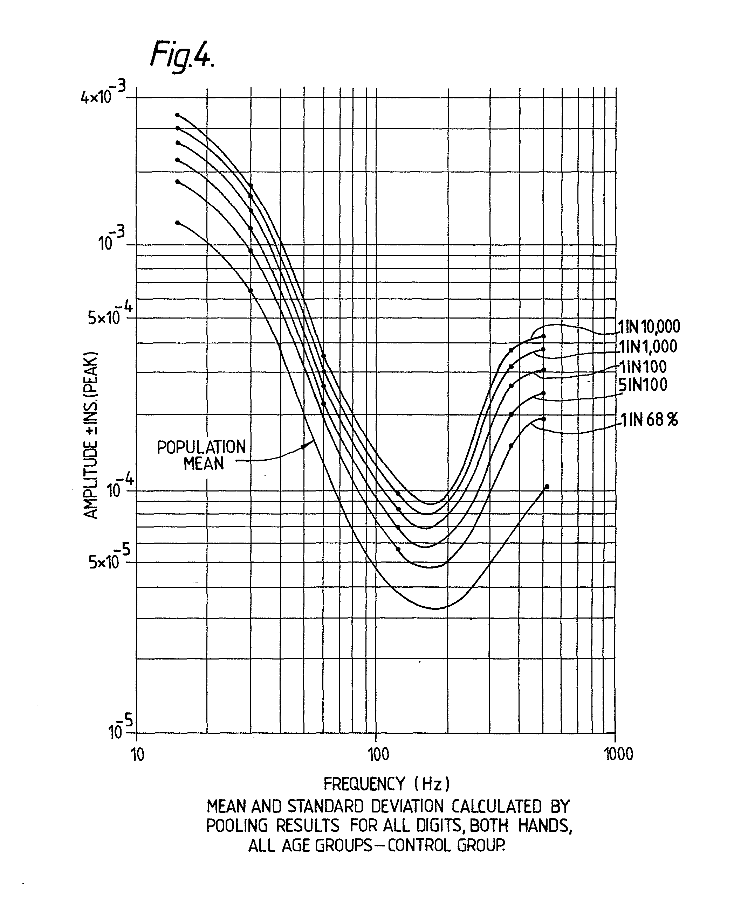

Referring now to Figure 4, there is shown the mean and standard deviations of responses to feeling vibrations calculated by pooling the results for all digits of both hands for persons of all age groups assuming normal distribution. From Figure 4 it will be seen that, in general, most people are more sensitive to vibration in the range of 100 Hz to 250 Hz than in the higher and lower frequencies.

-8-

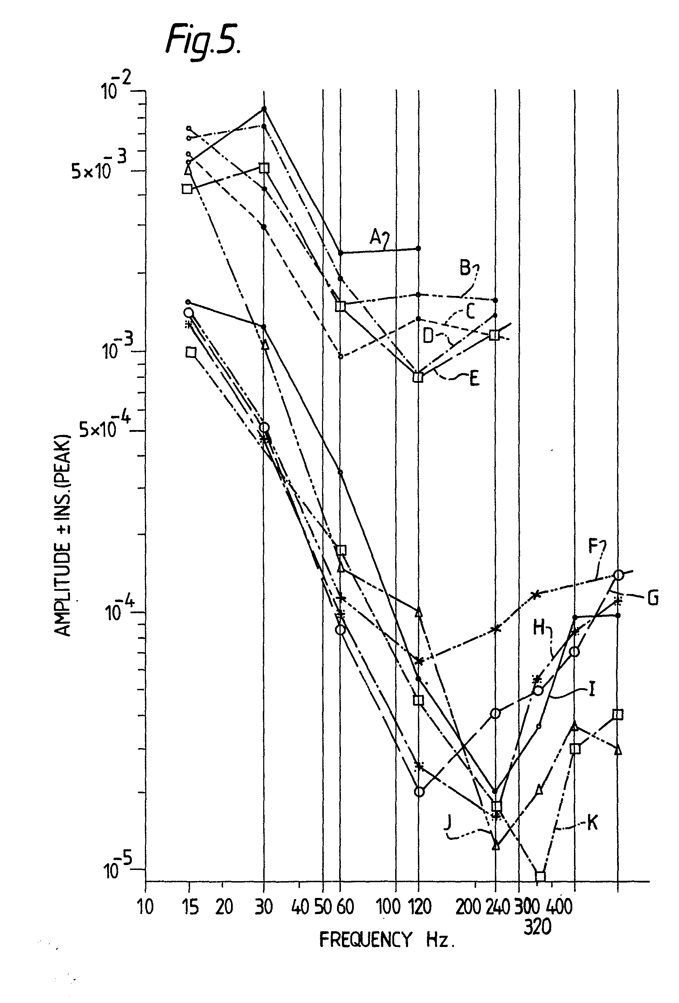

Referring to Figure 5 it will be seen that there is a marked difference in the levels of sensitivities for all frequencies between those known to suffer from vibration white finger, or Raynaud's Disease (as shown by graphs A to E ), compared with persons who do not suffer from V.W.F. or Raynaud's Disease. It will also be appreciated that the graphs F to K of those subjects not suffering from V.W.F. follow more closely the general trend indicated in Figure 4 whereas those prone to contract V.W.F. are noticeably different from the general trends.

The selection of frequency may be automated by cyclicly switching electronically to different frequency channels in descending or ascending order. Furthermore, it is not necessary to operate the apparatus on all of the frequency bands mentioned above. For example, to simplify the apparatus, it has been found that frequencies of say 90 Hz, 120 Hz, 240 Hz and 400 Hz will provide adequate information to determine cutaneous sensitivity.

## CLAIMS

1.  Apparatus for monitoring the cutaneous sensitivity of a person, characterised in that the apparatus comprises a vibratable member 14 against which the person places a part of their anatomy the cutaneous sensitivity of which is to be monitored, a vibrator means 12 for vibrating the member 14 at a preselected frequency, control means 25,26,28,29 for varying the amplitude of vibration of the member, and output means 30,31,32,36 for producing an output signal indicative of the threshold amplitude at which the person can feel the vibrations of the member 14.

2.  Apparatus according to Claim 1 wherein a frequency control means 24,29 is provided for selectively varying the frequency of vibration of the member 14.

3.  Apparatus according to Claim 1 or Claim 2 wherein the frequency control means 24,29 is operable to cause the member 14 to vibrate at one of a number of preselected frequencies in a range of frequencies.

4.  Apparatus according to any one of Claims 1 to 3 wherein the control means 24,29 is operable to increase gradually the amplitude of vibration of the member 14 from a predetermined value.

5.  Apparatus according to any one of the preceding claims wherein the control means 24,29 is operable to decrease gradually the amplitude of vibration of the member 14 from a predetermined value.

0072255

-10-

6.    Apparatus according to Claim 4 or Claim 5 wherein the control means 24,29 is operable to increase and decrease sequentially the amplitude of vibration of the member 14 relative to a predetermined value.

7.    Apparatus according to any one of the preceding claims wherein the member 14 is mounted on the core of a magneto-strictive transducer.

8.    Apparatus according to any one of the preceding claims wherein a means 11 is provided for controlling the force applied by the person on the member 14.

9.    Apparatus according to Claim 8 wherein the means 11 for controlling the force applied to the member 14 comprises a balanced beam 11,18 on which the vibrator means 12,13 and the member 14 is mounted and an indicator means 16,17,19,21 is provided to indicate when a predetermined force is applied to the member 14.

*Fig1.*

Fig.2.

# Fig.3.

f1 TO f8

Fig.4.

MEAN AND STANDARD DEVIATION CALCULATED BY
POOLING RESULTS FOR ALL DIGITS, BOTH HANDS,
ALL AGE GROUPS—CONTROL GROUP.

Fig.5.